# EUROPEAN PATENT APPLICATION

(11) **EP 4 438 096 A1**
(43) Date of publication of application: **02.10.2024**
(21) Application number: 23165453.4
(22) Date of filing: 30.03.2023
(51) Int. Cl.: A61M 25/00, A61M 25/09

(54) **MEDICAL SYSTEM FOR CONTROLLING DEVICE SYSTEM INNER DEVICE**

(71) Applicant: Koninklijke Philips N.V., 5656 AG Eindhoven (NL)
(72) Inventor: BALICKI, Marcin A., Eindhoven (NL); FOTOUHI, Javad, Eindhoven (NL); PATRICIU, Alexandru, 5656AG Eindhoven (NL); KYNE, Sean, Eindhoven (NL)
(74) Representative: Philips Intellectual Property & Standards

(57) **Abstract**

A catheter comprising an outer device 260 within which an inner device 250 can move in a lengthwise direction relative to the outer device. At least one of the inner device or outer device are moved according to a predetermined condition, the movement being controlled by a controller 210, the predetermined condition relating to one or more of a blood parameter of blood proximal to a distal end of the device system, a predetermined lack of mobility of the device system; and the location of the distal end of the outer device within the vascular structure.

## Description

### FIELD OF THE INVENTION

The present invention relates to the field of medical systems and controllers, and in particular to the provision of medical systems and controllers for controlling the movement of inner and/or outer devices of a device system such as a catheter, to reduce the risk of blood clot formation within the device system.

### BACKGROUND OF THE INVENTION

A device system such as a catheter, or any other coaxial elongated device or instrument adapted for use in vascular structures, may comprise an outer device and an inner device (e.g., a guidewire) than can move lengthwise relative to the outer device and can be at least partly translated within this outer device.

Catheter-related thrombosis is a relatively common complication of central venous catheter insertion. In these cases, clots may form inside the catheter cavity during inactivity. Therefore, when the guidewire travels upstream inside the catheter, it can push out the intraluminal clot. The interventionalists are burdened with constantly keep the tip of the guidewire near the tip of the catheter to reduce the chance of this complication.

### SUMMARY OF THE INVENTION

It is hereby proposed embodiments as recited in the claims.

A particular embodiment proposes automatic methods to control the inner device (such as a guidewire) and/or the outer device of device system such as a catheter such that the likelihood of clot-forming is reduced. The embodiment allows the inner device and/or outer device to be automatically controlled, responsive to a predetermined condition, so that it may be moved before a clot is formed, or to prevent a clot from spreading through the body of a patient. The predetermined condition relates to one or more of: a blood parameter of blood proximal to a distal end of the device system; a predetermined time period; and a position of the outer device inside a body of a patient.

According to another exemplary embodiment, there is provided a medical system for controlling a device system within a blood vascular structure, the device system comprising an elongated outer device and an elongated inner device, the inner device being moveable within the outer device, the medical system comprising a controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined lack of mobility of the device system; and blood flow in the area of the vascular structure where the distal end of the device system is located.

According to exemplary embodiments, there is provided a medical system for controlling a device system within a blood vascular structure, the device system comprising an elongated outer device and an elongated inner device, the inner device being moveable within the outer device, the medical system comprising a controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to clot forming at or proximate a distal end of the device system.

In an embodiment, the predetermined condition relating to clot forming relates also to one or more of: a blood parameter of blood proximal to a distal end of the outer device; a predetermined lack of mobility of the device system; and the location of the distal end of the outer device within the vascular structure.

In an embodiment, the controller is further configured to control the movement of the at least one of the inner device or the outer device to reduce a volume of blood contained between the distal end of the outer device and the distal end of the inner device.

In an embodiment, the predetermined lack of mobility is calculated according to a determined probability of a blood clot forming within or proximal to the distal end of the outer device within a defined time period.

In an embodiment, the determined probability comprises an assessment of one or more of the following factors: a time period since the distal ends of the inner and outer devices were last moved relative to each other; the volume of blood contained between the distal end of the outer device and the distal end of the inner device; a blood condition of the volume of blood; a blood condition of a second volume of blood external to the outer device; and a blood coagulation rate.

In an embodiment, the blood coagulation rate is determined according to one or more of: a separation distance between the distal ends of the inner and outer devices; and the volume of blood contained between the distal end of the outer device and the distal end of the inner device.

In an embodiment, the predetermined condition relating to a predetermined lack of mobility of the device system comprises one or more of: a relative positioning of the distal end of the inner device with respect to the distal end of the outer device not changing beyond a positioning change value above an associated predetermined time period; and the volume of blood contained between the distal end of the outer device and the distal end of the inner device not changing beyond a volume change value above an associated predetermined time period; a predetermined lack of motion of the outer device depending on the location of the outer device with respect to proximate anatomical features.

In an embodiment, said predetermined condition relating to a blood parameter of blood comprises one or more of: a measured or assessed change of blood property beyond a threshold value of blood property change; the volume of blood contained between the distal end of the outer device and the distal end of the inner device over time not changing enough to prevent a change of blood property beyond a predetermined level of blood property change; and a relative positioning over time of a distal end of the inner device with respect to a distal end of the outer device not changing enough to prevent a change of blood property beyond a predetermined level of blood property change.

In an embodiment, the controller is further configured to oscillate the position of the inner device or the outer device between a first position and a second position.

In an embodiment, the controller is further configured to control the movement of the at least one of the inner device and the outer device between a first position of the inner device with respect to the outer device in which the distal end of the inner device is inside the outer device, and a second position of the inner device with respect to the outer device, in which the inner device is in a more or less retracted position in the second position than in the first position.

In an embodiment, the controller is configured to identify at least one of a position or a movement of at least one of the inner device and the outer device, according to received images comprising at least at least a portion of the device system, and wherein the controller is configured to control said movement of the at least one of the inner device and the outer device according to the identified position or movement.

In an embodiment, the medical system is further configured to receive an image comprising at least a portion of the device system and to identify blood clot formation in the image, and wherein the controller is configured to control the movement of at least one of the inner device or the outer device if such blood clot formation fulfills the predetermined condition relating to said blood parameter of blood.

In an embodiment, the predetermined condition relating to the blood flow in the area of the vascular structure where the distal end of the device system is located depends on the predetermined lack of motion of the outer device at this location and/or to the distance between the measured or assessed distal end of the outer device with a proximate vessel wall.

According to another embodiment, there is provided a method of controlling a device system within a blood vasculature structure, the device system comprising an outer device having a lengthwise direction and an inner device positioned inside the outer device, the method comprising the steps of: determining if a predetermined condition has been met, wherein the predetermined condition relates to at least one of: a blood parameter of blood proximal to a distal end of the device system; a predetermined lack of mobility of the device system; and the location of the distal end of the outer device within the vascular structure; and controlling the movement of at least one of the inner device or outer device responsive to the determination that a predetermined condition has been met.

In an embodiment, the movement of at least one of the inner device or outer device responsive to the determination that a predetermined condition has been met comprises controlling the movement of the at least one of the inner device or the outer device to reduce a volume of blood contained between the distal end of the outer device and the distal end of the inner device.

In an embodiment, the predetermined time period is calculated according to a determined probability of a blood clot forming within the distal end of the outer device within a defined time period. In a further embodiment, the determined probability comprises an assessment of one or more of the following factors: a time period since the distal ends of the inner and outer devices were last moved relative to each other; the volume of blood contained between the distal end of the outer device and the distal end of the inner device; a blood condition of the volume of blood contained between the distal end of the outer device and the distal end of the inner device; a blood condition of a second volume of blood external to the outer device; and a blood coagulation rate. In a yet further embodiment, the blood coagulation rate is determined according to one or more of: a separation distance between the distal ends of the inner and outer devices; and the volume of blood contained between the distal end of the outer device and the distal end of the inner device.

In an embodiment, the predetermined condition relating to a predetermined lack of mobility of the device system comprises one or more of: a relative positioning of a distal end inner device with respect to a distal end of the outer device which not changing beyond a positioning change value above the predetermined time period; the volume of blood not changing beyond a volume change value above the associated predetermined time period; a predetermined lack of motion of the outer device depending on the location of the outer device with respect to proximate anatomical features.

In an embodiment, the predetermined condition relating to a blood parameter of blood proximal to a distal end of the device system comprises one or more of: a measured or assessed change of blood property beyond a threshold value of blood property change; the volume of blood contained between the distal end of the outer device and the distal end of the inner device over time not changing enough to prevent a change of blood property beyond a predetermined level of blood property change; and a relative positioning over time of a distal end of the inner device with respect to a distal end of the outer device not changing enough to prevent a change of blood property beyond a predetermined level of blood property change.

In an embodiment, the controlling the movement of the at least one of the inner device or the outer device comprises oscillating the position of the inner device or the outer device between a first position and a second position.

According to another embodiment, there is provided a computer program product comprising computer program code means which, when executed on a computing device having a processing system, cause a processing system to perform all of the steps of the method steps of any herein described methods or to run a controller of any herein described medical system.

According to yet another embodiment there is provided a controller for use in a medical system for controlling a device system for use in blood vessels, the device system comprising an elongated outer device and an elongated inner device, the inner device being moveable within the outer device, the controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined lack of mobility of the device system; and blood flow in the area of the vascular structure where the distal end of the device system is located.
controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined time period; and a position of the outer device inside a body of a patient.

According to exemplary embodiments, there is provided a medical system for controlling a device system for use in blood vessels, the device system comprising an elongated outer device and an elongated inner device, the inner device being moveable within the outer device, the medical system comprising a controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined time period; and a predetermined lack of motion of the outer device.

According to exemplary embodiments, there is provided a medical system for controlling a device system within a blood vascular structure, the device system comprising an elongated outer device and an elongated inner device, the inner device being moveable within the outer device, the medical system comprising a controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined time period; and a position of the outer device inside a body of a patient.

The present disclosure provides a system for reducing the risk of a clot forming within a device system such as a catheter. By moving the inner guidewire relative to the outer device, or the outer device relative to the inner device, clots may be prevented from forming, or clots that have formed may be removed.

At least one of the inner device and outer device are moved. At least one of the inner guidewire and outer device is configured to move backwards and forwards in the lengthwise direction, relative to the other. This means that either the inner guidewire is configured to move relative to the outer device, the outer device is configured to move relative to the inner guidewire, or both are configured to move relative to each other. Embodiments propose various approaches to controlling the motion of the inner guidewire or the outer device relative to the other. In embodiments, both are moved.

Automatically controlling the movement of the inner guidewire and/or the outer device reduces the burden on interventionalists who may otherwise need to manually perform this movement to prevent clot formation.

In the context of the present disclosure, the catheter may not be entirely straight and may comprise bends. Accordingly, the precise orientation of the lengthwise direction may vary along its length. Lengthwise direction means the general direction along the catheter's length, between its ends. In use the inner guidewire may be rotated whilst being translated, especially when is diameter is much smaller than the inner diameter of the outer device.

Automatically controlling the movement of the inner guidewire allows the guidewire to be moved prior to a clot having time to form.

The inner device such as a guidewire (or the outer device) may be moved more than once, as necessary to prevent clot formation. The inner device may be moved continuously. The inner device may be moved periodically, i.e., repeatedly moved and then moved again a time period later. Such movement may also be referred to as intermittent movement or continual movement (i.e., repeatedly but not continuously).

The determination of whether a predetermined condition has been met may comprise obtaining or determining an operating parameter of the device system. The predetermined condition may relate to one or more of: a volume of blood between the tips of the inner guidewire and the outer device; a distance between a tip of the inner guidewire and a corresponding tip of the outer device; a time period since the tips of the inner guidewire and the outer device last moved relative to each other; the location of the devices in the body. Obtaining comprises measuring or receiving, e.g., via an input.

The predetermined lack of motion of the device system can be determined by the device system being located inside a region of the body for a given time, or by an amount of displacement imparted by the robot on the device over a period of time. This can be measured in x-ray images (period snapshots) and tracking using common computer vision methods or Al methods like UNet. The robot tracked version may be more robust as it does not require x-ray, and uses encoder information to establish the displacement amount over time. The amount of displacement that constitutes motion can be empirical, and depends on anatomical location of the device. In an embodiment, the predetermined lack of motion comprises a lack of motion of the inner or the outer of more than 10mm within a predetermined time period.

The predetermined lack of motion of the device system may comprise a predetermined lack of motion of the outer device with respect to a body when the device system is inserted in a body.

The volume of blood contained between the distal ends of the inner and outer devices may be determined from the relative distance between the distal ends of the inner device and the outer device, and the internal diameter of the external device along its length.

In a further variation, determining when the volume has reduced below a threshold allows it to be determined that any clot has been removed or prevented from forming, and that the blood within the end of the catheter has been expelled. Then, for example, the inner device may be moved back to a retracted position, drawing fresh blood into the end of the catheter. Accordingly, the blood within the end of the device system may be refreshed to prevent a clot from forming.

Furthermore, other, secondary, factors may be taken into consideration in determining an appropriate time period within which one of the inner and outer devices needs to be moved. These factors include may include one ore more of: patient status e.g., types of drugs used in the blood stream, which may affect likelihood of clot formation; or prior events (stroke) and treatment that could increase blood cell damage that leads to formation of clots.

The risk of a clot forming may depend on the position of the medical device within the body. Thus, the location of the device system in the body may be relevant, since, for example, the risk of clot formation in an artery is more dangerous than in a vein, or the device system may be located to an injury site where coagulation risk may increase. Furthermore, proximity to other pathology such as atherosclerosis may affect likelihood of clot formation. Indeed, low blood flow areas in the vasculature contribute to increased stroke risk, such as areas in more distal vessels (vs aorta), and in situations where the device system (e.g., catheter) tip is near the edge of the vessels.

The inner device and/or outer device may be moved responsive to a pre-determined time period having elapsed. For example, the inner device may be moved automatically 10s, 60s, or 120s after it was last moved. These periods are examples only, and it may be moved after any predetermined time period. Automatically moving the inner or outer device according to a time period elapsing means the system may reliably prevent clots from forming, without a need for human intervention. Accordingly, the medical system may be more reliable and may reduce human resources required to operate it.

The predetermined time period may be a time period since the device was turned on, or may be a time period since the device was inserted inside a body of a patient. The predetermined time period may be zero. For example, the inner and/or outer device may be moved periodically or continuously once the outer device has been positioned inside the body of a patient. The predetermined time period may be a maximum length time the outer device may be allowed to remain stationary within the body of a patient without moving. For example, if the medical device (e.g., a catheter) is determined to have been static (i.e., not moving) relative to the patient for N amount of time (i.e., when it has been static for longer than a predetermined time period), it is more likely that a clot may develop. Thus, the inner device and/or outer device may be moved accordingly, to reduce the risk of a clot forming. Such movement may additionally overcome a problem with a saline drip line that is attached to the medical device could malfunction or run out, which decreases lubrication inside the catheter or creates a vacuum that causes blood to go back up the medical device. This problem may be prevented by periodic movement.

The blood parameter of blood proximal to a distal end of the device system may comprise a measured or derived characteristic of the blood, e.g., a blood condition. The blood parameter of blood proximal to a distal end of the device system may comprise a blood condition relating to blood within the device. Alternatively, the parameter may relate to blood on the outside of the device system, e.g., a blood condition relating to the blood outside the device system, near to the tip (the distal end) of the outer device. A clot forming here may have initially been formed on the inside of the outer device. Accordingly, if a clot is determined outside the medical device, the inner device may be retracted relative to the outer device to cause a vacuum within the outer device which may draw blood and the clot into the device system, after which the device system may be withdrawn from the body and the clot dealt with, e.g., disposed of.

The blood parameter may relate to a blood condition. For example, the A blood condition may relate to one or more of a volumetric, geometric or a quality condition of the blood, contained within the device system or proximal to the end of the device system. For example, the volumetric condition may be a volume of blood enclosed between walls of the outer device and an end of the inner device. For example, a geometric condition may be a shape of blood enclosed between walls of the outer device and an end of the inner device, and may comprise a relative length to a relative diameter or width. A quality condition may be a measured condition of the blood, i.e., relating to a physical characteristic of the blood, such as viscosity.

The predetermined condition may relate to one or more of: a blood parameter of blood proximal to a distal end of the device system meeting a condition, or exceeding or not exceeding a threshold value; a predetermined time period exceeding a threshold value or falling within a predetermined range of time values; and a position of the outer device inside a body of a patient meeting or not meeting one or more criteria.

A threshold value associated with the predetermined condition may be set according to clot formation rate data.

In an embodiment the controller may be configured to determine a location of each of the distal ends of the inner and outer devices to determine a separation of the distal ends of the inner and outer devices. In a further embodiment, the separation may be sued to determine a volume of blood between the distal ends.

In an embodiment, the controller is further configured to control the movement of the at least one of the inner device or the outer device to reduce a volume of blood contained between the distal end of the outer device and the distal end of the inner device.

The volume of blood may be the volume of blood contained within the outer device, between the distal ends of the inner and outer devices.

The reduction of volume may comprise determination of when the volume has reduced below a threshold. This allows it to be determined that any clot has been removed or prevented from forming, and that the blood within the end of the catheter has been expelled.

Accordingly, all or some of the blood within the device system may be pushed out of the device system, and the risk of clot formation may be reduced. The act of moving the blood may reduce the risk of clot formation.

In a further variation, the inner guidewire instead may be moved to a more retracted position, or back to previous position, drawing fresh blood into the end of the device system. Accordingly, the blood within the end of the device system may be refreshed to prevent a clot from forming or to draw a clot external to the device system into the device system to prevent it travelling within the body of a patient.

In a further embodiment, the controller is further configured to control the movement of the at least one of the inner device or the outer device to reduce a volume of blood contained between the distal end of the outer device and the distal end of the inner device below a threshold volume.

In an alternative embodiment, the controller is further configured to control the movement of the at least one of the inner device or the outer device to minimize the separation between the two, wherein the minimization is to appropriate tolerance or range close to zero. Accordingly, the risk of clot formation may be reduced.

In an embodiment, the predetermined time period is calculated according to a determined probability of a blood clot forming within or proximal to the distal end of the outer device within a defined time period.

Assessing the probability of clot formation within or proximal to the distal end of the outer device within a defined time period allows the inner and/or outer device to be moved at an appropriate time to reduce the risk of a clot forming. Accordingly, it may be moved no more than necessary, but as often as required to minimize the clot formation risk to an appropriate level.

A plurality of determined probabilities may be calculated, each according to a different time period. Thus, an appropriate time to move the inner and/or outer may be determined according to the risk of clot formation within each of a plurality of time periods. For example, it may be determined that the risk of clot formation within a first time period, A seconds, may be X %, and the risk of clot formation within a second time period, B seconds, may be Y %. X may be higher than Y. Thus, it may then be determined that the inner and/or outer should be moved within B seconds, due to the unacceptable risk level associated with not moving the inner and outer for A seconds.

In an embodiment, the determined probability comprises an assessment of one or more of the following factors: a time period since the distal ends of the inner and outer devices were last moved relative to each other; the volume of blood; a measured blood condition of the volume of blood; a measured blood condition of a second volume of blood external to the outer device; and a measured or determined blood coagulation rate of the volume of blood.

Accordingly, an appropriate time to move the inner and/or outer may be determined.

The time since the distal ends of the inner and outer were last moved relative to each other may affect the rate at which a clot may form and thus this time period may be incorporated into the determined probability. If the distal ends have not been moved relative to each other for a while, there is a greater risk of clot formation according to the time since they were last moved. Accordingly, the time since they were last moved relative to each other may be determined and one or both of the inner and outer device may be moved accordingly.

The volume of blood enclosed between the distal ends of the inner and the outer may affect the rate at which a clot may form, and thus the volume may be incorporated into the determined probability.

A measured blood condition of the volume of blood enclosed between the distal ends of the inner and the outer may affect the rate at which a clot may form, and thus the measured blood condition may be incorporated into the determined probability.

A measured blood condition of a second volume of blood external to the outer device may affect the rate at which a clot may form, and thus the measured blood condition may be incorporated into the determined probability.

A measured or determined blood coagulation rate of the volume of blood may affect the rate at which a clot may form, and thus the measured or determined blood coagulation rate may be incorporated into the determined probability.

In an embodiment, the measured or determined blood coagulation rate is determined according to one or more of: a separation distance between the distal ends of the inner and outer devices; and the volume of blood. The separation distance and/or the volume of the blood may affect the blood coagulation rate, and thus one or both may be incorporated into the determined probability.

In an embodiment, there is provided a medical system for controlling a device system for use in blood vessels, the device system comprising an elongated outer device and an elongated inner device, the inner device being moveable within the outer device, the medical system comprising a controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined time period; a position of the outer device inside a body of a patient; and a separation distance between distal ends of the inner and outer devices; and the controller is further configured to control the movement of the at least one of the inner device or the outer device to reduce a volume of blood contained between the distal end of the outer device and the distal end of the inner device.

Reducing the volume of blood according to the separation distance of the distal ends means that the blood may be pushed out of the device system and the risk of clot formation may be reduced, when the separation distance exceeds a threshold value. The reduction in the volume of blood may be responsive to both the separation distance and the time period since the distal ends were last moved relative to each other. Accordingly, the separation distance and time period since last movement may be determined, and the volume of blood automatically reduced if both the time period exceeds a threshold time period and the separation distance exceeds a threshold separation distance. The reduction may be a predetermined reduction, e.g., according to a fixed or proportional reduction in the separation distance, or a fixed or proportional reduction in the associated enclosed volume of blood between the distal ends according to the separation distance, or the reduction may be a minimization to reduce the volume of blood to a value close to zero, or less than a threshold volume close to zero.

In an embodiment, the predetermined condition relates to one or more of: a relative positioning of the distal end of the inner device with respect to the distal end of the outer device which does not change beyond a positioning change value above an associated temporal value; a change of blood property beyond a threshold value of blood property change; the volume of blood not changing beyond a volume change value above an associated temporal value; the volume of blood over time which not changing enough to prevent a change of blood property beyond a predetermined level of blood property change; and a relative positioning over time of a distal end of the inner device with respect to a distal end of the outer device not changing enough to prevent a change of blood property beyond a predetermined level of blood property change.

In an embodiment, the controller is further configured to, for one or more oscillation, oscillate the position of the inner device or the outer device between a first position relative to the other and a second position relative to the other.

For example, the controller may move (i.e., oscillate) the inner device between a first position and a second position, for at least one oscillation, causing an associated oscillation of the separation distance of the inner and outer device distal ends.

The inner and /or outer device may each be controlled to move a respective number of oscillations, for example, 1, 2, 3, 4, 5, 6, 7, 8, 9, 10, 20, 30, 50, 100 or 200.

The oscillations may be continuous, or may be continual (i.e., repeated indefinitely but with pauses between each oscillation)

Alternatively, the controller may move (i.e., oscillate) the inner device between a first position and a second position, for at least one oscillation, causing an associated oscillation of the separation distance of the inner and outer device distal ends.

Alternatively, the controller may move (i.e., oscillate) both the inner device between a first position and a second position, and the outer device between a respective first position and respective second position, for at least one oscillation each. The movement of the inner device and outer device may be sequential or at the same time. The movement of the inner device and outer device may further be at the same rate or at different rates. The movement of one or both of the inner and outer devices may be continuous, or intermittent. An oscillation cycle means a movement from a first position to a second position and back again.

The inner and/or outer devices may continuously vibrate between a first and second position, with a fixed vibration frequency. The vibration frequency is defined as the number of times the inner and/or outer devices is in or passes through a particular position in one second, wherein the particular position is between the first and second positions (inclusive of the first and second positions).

In an embodiment, the medical system comprises comprising a separation distance between the distal ends of the inner and outer devices, and wherein the controller is further configured to, for at least one oscillation, oscillate at least one of the inner device and the outer device between a first position that relates to a first separation distance, and a second position that relates to a second separation distance, responsive to the predetermined condition. The predetermined condition may comprise the device being positioned inside a human body and a time period having elapsed since the device has been positioned or last moved within the body. The predetermined condition may comprise the device being positioned inside a human body and the predetermined time period may be zero, i.e., the inner and/or outer may be continuously oscillated as soon as the device system is positioned inside a body, or until a signal is received to stop oscillating. One or more of the position of the device within the body, the volume of blood contained in the distal end of the outer (between the distal end of the inner device and the distal end of the outer device), and the separation distance of the distal ends may be used by the controller to affect one or more of the speed of oscillation, the number of oscillations, whether the inner and/or outer is moved, and (when the oscillations are intermittent) the time period between oscillations.

Oscillating the inner device or the outer device between the first and second positions may be particularly effective at preventing clots from forming.

In an embodiment, the controller is further configured to control the movement of the at least one of the inner device and the outer device between a first position of the inner device with respect to the outer device in which the distal end of the inner device is inside the outer device, and a second position of the inner device with respect to the outer device, in which the inner device is in a more retracted position in the first position than in the second position.

The second position may be wherein the inner is a retracted within the outer device, but less retracted than the first position (i.e., such that the inner device distal end is contained within outer device device), or the second position may be a position in which the inner device extends outside the outer device.

The first position may be a retracted position, and the second position may be a less retracted position, such as where the distal ends are aligned. Alternatively, the second position may be a position in which the guidewire extends beyond the end of the outer device. The second position may be a position in which the distal ends of the inner device and the outer device are aligned - i.e., wherein the inner guidewire is neither retracted nor extending beyond the outer device.

It may be useful to retain the inner device in a first, retracted position when not in use. In this position, the end of the guidewire is removed from the end of the guidewire, and the guidewire is entirely contained within the end of the outer device. This leaves a volume of blood within the device system.

If the guidewire is retained in this first position for an extended period, a clot may form within the end of the device system. Accordingly, by moving the guidewire to a second, less retracted, position for a period, clot formation within the device system may be minimized.

This second position may be closer to the end of the device system than the first position, but in a position in which the inner device remains still fully enclosed within the end of the catheter.

This second position may be adjacent to the opening in the outer device, i.e., such that the distal ends of the inner device and of the outer device are aligned. This second position be a position in which the inner device protrudes from the end of the outer device.

Accordingly, the movement between the first and second position may move reduce the volume of blood contained within the distal end of the outer device or may substantially remove all of the blood from the outer device. In both cases, the risk of clot formation within the outer device may be reduced.

In an embodiment, controller is configured to identify at least one of a position or a movement of at least one of the inner device and the outer device, according to images comprising at least at least a portion of the device system, and wherein the controller is configured to control said movement of the at least one of the inner device and the outer device according to the identified position or movement.

The image may comprise x ray images. X ray images may provide a convenient or accurate method to determine the relative position of the inner and outer devices.

In an alternative embodiment, the medical system comprises a robot encoder that calculates the position of at least one of the inner device and or outer device according to the control signals that have been sent by the controller. Accordingly, the position of the inner and outer device may be determined, and the relative positions may be determined.

According to an embodiment, there is provided a method of controlling a device system for use in blood vessels, the device system comprising an outer device having a lengthwise direction and an inner device positioned inside the outer device, the method comprising the steps of: determining if a predetermined condition has been met, wherein the predetermined condition relates to at least one of: relates to one or more of: a blood parameter of blood proximal to a distal end of the device system; a predetermined time period; and a position of the outer device inside a body of a patient; and controlling the movement of at least one of the inner device or outer device responsive to the determination that a predetermined condition has been met.

In an embodiment, the method comprises a further position determination step wherein the relative position of each of the distal ends of the inner and outer devices is determined.

In an embodiment, the controller is further configured to control the movement of the at least one of the inner device or the outer device to reduce a volume of blood contained between the distal end of the outer device and the distal end of the inner device.

In an embodiment, the predetermined time period is calculated according to a determined probability of a blood clot forming within the distal end of the outer device within a defined time period, and

In an embodiment, the determined probability comprises an assessment of one or more of the following factors: a time period since the distal ends of the inner and outer devices were last moved relative to each other; the volume of blood; a measured blood condition of the volume of blood; a measured blood condition of a second volume of blood external to the outer device; and a measured or determined blood coagulation rate of the volume of blood.

In an embodiment, the measured or determined blood coagulation rate is determined according to one or more of: a separation distance between the distal ends of the inner and outer devices; and the volume of blood.

In an embodiment, the predetermined condition relates to one or more of: a relative positioning of a distal end inner device with respect to a distal end of the outer device which does not change beyond a positioning change value above an associated temporal value; a change of blood property beyond a threshold value of blood property change; the volume of blood not changing beyond a volume change value above an associated temporal value; the volume of blood over time which not changing enough to prevent a change of blood property beyond a predetermined level of blood property change; and a relative positioning over time of the distal end of the inner device with respect to the distal end of the outer device not changing enough to prevent a change of blood property beyond a predetermined level of blood property change.

In an embodiment, the controlling the movement of the at least one of the inner device or the outer device comprises, for one or more oscillation, oscillating the position of the inner device or the outer device between a first position relative to the other and a second position relative to the other.

According to yet another embodiment there is provided a controller for use in a medical system for controlling a device system for use in blood vessels, the device system comprising an elongated outer device and an elongated inner device, the inner device being moveable within the outer device, the controller configured to control the movement of at least one of the inner device or the outer device in the lengthwise direction relative to the other, wherein the controller is further configured to control the movement of the at least one of the inner device or the outer device responsive to a predetermined condition, and wherein the predetermined condition relates to one or more of: a blood parameter of blood proximal to a distal end of the device system; a predetermined time period; and a position of the outer device inside a body of a patient.

In an embodiment there is provided a medical system for controlling a device system for use in blood vessels, the device system comprising an elongated outer device and an elongated inner device, the inner device being moveable within the outer device, the medical system comprising a controller configured to control the movement of at least one of the inner device or the outer device in the lengthwise direction relative to the other, wherein the controller is further configured to control the movement of the at least one of the inner device or the outer device responsive to a predetermined condition to reduce a volume of blood enclosed between a distal end of the inner device and a distal end of the outer device, wherein the predetermined condition relates to one or more of: a separation distance between the distal end of the inner device and the distal end of the outer device; a blood parameter of blood proximal to a distal end of the device system; a predetermined time period; and a position of the outer device inside a body of a patient.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 illustrates how a clot may form within the known catheter comprising an outer device and an inner device;
Fig. 2 illustrates a method of controlling a catheter to prevent a clot forming;
Fig. 3 illustrates a system for controlling a catheter to prevent a clot forming;
Fig. 4 illustrates how imaging data can be used to control the position of the inner guidewire to prevent a clot from forming in a catheter; and
Fig. 5 is a simplified block diagram of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. If the term "adapted to" is used in the claims or description, it is noted the term "adapted to" is intended to be equivalent to the term "configured to".

It should be understood that the Figures are merely schematic and are not drawn to scale. It should also be understood that the same reference numerals are used throughout the Figures to indicate the same or similar parts.

Proposed concepts thus aim to provide schemes, solutions, concepts, designs, methods and systems pertaining to reducing the risk of a clot forming in a catheter.

By way of example only, illustrative embodiments may be utilized in many different types of clinical, medical or subject-related environments, such as a hospital, doctor's office, medical-research facility, ward, care home, person's home, etc.

Proposed is an approach for controlling a device system for use in blood vessels, the device system comprising an outer device having a lengthwise direction and an inner device positioned inside the outer device, comprising: determining if a predetermined condition has been met, wherein the predetermined condition relates to at least one of: a blood parameter of blood proximal to a distal end of the device system; a predetermined time period; and a position of the outer device inside a body of a patient; and controlling the movement of at least one of the inner device or outer device responsive to the determination that a predetermined condition has been met.

In the scenario where the distal end of the inner device is contained within the outer device, the monitoring of a predetermined condition may relate to the volume of blood contained between the distal ends of the inner and outer devices. Movement of the inner device may be initiated if said volume stays in a predetermined range of volume for more than a predetermined time period. This movement of the inner device may reduce a volume of blood between the distal ends of the inner and outer devices, with the associated movement of blood causing reducing the risk of a clot forming in static blood.

In embodiments, the system is further arranged to control the inner device to go back to its initial position once said reduction of volume has been implemented. This movement and return movement may be repeated in immediate succession in the form of a back-and-forth movement.

In embodiments, an oscillation (which may be a singular, a continuous or an intermittent oscillation) of the inner device distal end between a first and second position may be implemented. The magnitude or frequency of the oscillation may change depending on said volume determined over a predetermined time period.

In embodiments, said predetermined range of volume over said predetermined limit of time period, is determined based on clot formation properties.

In an embodiment, the inner guidewire is advanced (moved towards the end of the catheter) to push blood out from the device. In an alternative method, the inner guidewire is retracted to move the blood by suction. This suction motion may cause fresh blood to rush into the end of the device system, which may prevent a clot from forming in the blood within the end of the outer device.

In the embodiments herein, a catheter comprising an outer device and inner guidewire is used to explain the invention. The invention can be used with any medical device system comprising and inner and outer device which can move relative to each other, and is not limited to use with a catheter. Similarly, the invention is not limited to use with an inner guidewire and other inner devices may be used.

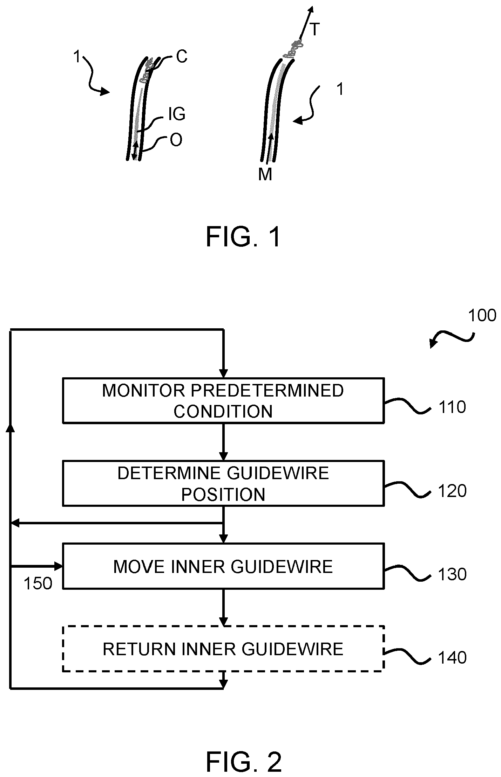

Fig. 1 illustrates how a clot may form within the known catheter 1. The left-hand side Figure shows a catheter 1 at a first time, and the right-hand side Figure shows that same catheter 1 at a second, later, time. The catheter 1 comprises and outer device O within which an inner guidewire IG can move lengthwise relative to the outer device O. Accordingly, a tip of the inner guidewire IG can be moved relative to a tip of the outer device O. The tip of the inner guidewire IG may be retracted compared to the tip of the outer device O, i.e., it may be entirely contained within the outer device O, the tip of the inner guidewire IG be aligned with the tip of the outer device OG, or the tip of the inner guidewire IG may be extended beyond the tip of the outer device.

When the tip of the inner guidewire IG is in the retracted position, a volume of blood is contained within the end of the catheter 1, contained in the volume between the distal ends of the inner guidewire IG and the outer device O. In this position, a clot C may form during a period of inactivity. This clot may be formed in only a few minutes.

If the distal end (i.e., the tip) of the inner guidewire IG is subsequently moved from the retracted position (as shown left) to a position towards the end of the catheter 1 (as shown right), after a stationary period during which time a clot has formed within the end of the catheter, the inner guidewire IG may push the clot out of the catheter 1, after which it may travel upstream in direction T. The clot C then travels through the vasculature potentially creating a life-threatening embolism, or a venous thrombosis.

Accordingly, in the prior art, an interventionalist need to continuously monitor the position of the inner guidewire tip IG_{TIP} relative to the outer device O tip, and moves the inner guidewire tip IG_{TIP} periodically to prevent a clot C from forming within the catheter 1. This is time consuming, and any failure to move the inner device IG in time may be life-threatening.

In the prior art, one way in which physicians mitigate potential clot formation is by flushing the catheter with saline. However, that requires the catheter to be empty of devices which is impractical in many cases. Another known solution is to arrange the inner device inside the catheter, so the tips are aligned, reducing the volume inside the catheter. However, this this requires attention to make sure the devices are not advertently disturbed, and this approach requires x-ray to monitor the location of the two device tips. In both cases, a human needs to frequently perform these interventions disturbing the workflow, increasing x-ray radiation, and most importantly potential human errors leading to severe complications.

To aid understanding of the proposed concept(s), an exemplary embodiment of a method for preventing clot formation within a catheter will now be described with reference to Fig. 2.

Fig. 2 illustrates a method 100 of controlling a catheter to prevent a clot forming, according to the present invention.

In a monitoring step 110, one or more predetermined condition is monitored, wherein the predetermined condition relates to at least one of: a blood parameter of blood proximal to a distal end of the device system; a predetermined time period; and a position of the outer device inside a body of a patient. In an exemplary embodiment, three predetermined conditions are monitored: a volume of blood enclosed in the distal end of the outer device (between the distal ends of the inner and outer device); a predetermined time period during which a clot my typically form; and a position of the outer device inside a body of a patient.

In a position determination step 120, the position of the inner guidewire IG relative to the outer device O may be determined. From this, a volume of blood between the tips may be derived, based on the geometry of the devices. In an embodiment, this step comprises determination of the position of the tip of the inner guidewire IG relative to tip of the outer device O, but alternatively the relative position or another part of the inner guidewire IG or another part of the outer device O may be determined.

Following monitoring step 110 and position determination step 120, an assessment is made of whether a movement of the inner and/or outer devices is necessary or useful to prevent a clot from forming. If movement is not required, monitoring step 110 and position determination step 120 are repeated until the assessment indicates that a movement is required.

In a movement step 130, the inner guidewire IG is moved relative to the outer device O, if the assessment indicates that movement may be necessary or useful.

The predetermined time period may be varied according to the position in the body and an associated assessment of the probability and clot formation in that part of the body and the associated risk of such a clot. For example, if the outer device is in an arterial position, the predetermined time period may be reduced due to the greater potential consequence of a clot in an artery compared to in a vein.

As a further example, if, in the monitoring step 110, it is determined that a time period that has elapsed since the inner guidewire last moved relative to the outer device is small, and below a predetermined threshold, then it may not be necessary to move the inner guidewire IG yet, and no movement may be required in movement step 130. Alternatively, in position determination step 120, it may be determined that the tip of the inner guidewire IG and the tip of the outer device O are aligned, and that accordingly it is not necessary to move the inner guidewire IG to prevent a clot from forming (since no volume of blood is contained within an end of the catheter), then no movement may be required in movement step 130, even if the pre-determined time period has been exceeded.

However, the information gathered in the monitoring step 110 and the position determination step 120 may indicate that is it necessary to move the inner guidewire relative to the outer device to prevent a clot from forming. For example, it may be determined in monitoring step 110 that the period since the inner guidewire IG was last moved has exceeded a threshold, and it may be determined in position determination step 120 that there is a gap between the tips of the inner guidewire and the outer device. This gap is used to derive a of blood between the tips, which exceeds a threshold. Due to the time period and volume of blood both exceeding a predetermined respective threshold, there may be a risk of a clot forming. The location indication further confirms that the device system is inside the body. Thus, the inner guidewire may then be moved to prevent a clot from forming within the end of the catheter.

In an embodiment, the predetermined condition relates only to blood parameter proximal to the end of the outer device, for example the volume of blood contained within the outer device. If this volume exceeds a threshold value, the inner is moved relative to the outer to reduce the volume of blood contained in the outer, thereby preventing a clot from forming. This movement may be further conditional on a pre-determined time period being exceeded, for example the volume threshold being exceeded for more the two minutes.

In an embodiment, the distance between the distal ends of the inner and outer may be assessed to determine if it exceeds a distance threshold. If it does, the volume between the distal ends may be reduced, optionally to below a target volume value. This movement may be further conditional on a pre-determined time period being exceeded, for example the distance threshold being exceeded for more the two minutes.

In an embodiment, monitoring step 110 and position determination step 120 may be combined in one step. For example, monitoring step may comprise monitoring a distance between the tips of the inner guidewire and the outer device, from which the volume between of blood between the tips can be derived, and the determination of the distance between the tips may also then provide the necessary information as to the inner guidewire position relative to the outer device, and a separate position determination step 120 may not be necessary.

In an embodiment, the position determination step 120 may comprise referring to a last known position of the guidewire. For example, the position to which a guidewire was moved in a previous movement step 130, according to a first cycle of the method, may provide the information for a subsequent determination step 130, according to a second cycle of the method.

In an embodiment, movement step 130 comprises more than one movement, e.g., a movement from a first position to a second position, and a further movement to a third position that is different to the first and second positions.

In an embodiment, movement step 130 comprises an oscillating movement of the inner guidewire tip between a first position and a second position. In a variation, the speed at which the inner guidewire tip moves between the positions is constant. In a further variation, the inner guidewire tip may accelerate as it leaves one of the positions to a maximum speed, and then decelerate as it approaches the other position. In a further variation a single oscillation (one back and forth motion) is undertaken during the movement step 130. In a further variation, the oscillation is continuous, i.e., non-stop movement between the first and second positions. In this embodiment the tip may be considered to continuously vibrate between the two positions with a defined vibration frequency. In a further alternative, a series of discrete oscillations may be undertaken, with a pause period between, i.e., the oscillations may be intermittent. In a further variation applicable to each of these variations, the outer device oscillates between the two positions. In a yet further variation, both the inner and outer guidewire oscillate between respective first and second positions.

In an embodiment, movement step 130 comprises movement of the at least one of the inner device and the outer device between a first position of the inner device with respect to the outer device in which the distal end of the inner device is inside the outer device, and a second position of the inner device with respect to the outer device, in which the inner device is in a more retracted position in the first position than in the second position. For example, the movement step moves the inner guidewire from a more retracted position to a less retracted position, wherein the tip of the inner is contained within the outer in both positions. In a further alternative, the movement step moves the inner guidewire from a retracted position where its tip is contained within the outer device, to an extended position where its tip extends beyond the tip of the outer device.

The method 100 comprises an optional return step 140, wherein the inner guidewire is returned to the position that was determined in position determination step 120, or to a different position again.

The method 100 comprises an optional loop 150, wherein the movement step and the optional return step 140 are repeated as many times as determined by a controller. For example, the loop may require that the movement step 130 and return step 140 are repeated twice, prior to the monitoring step of a subsequent cycle of the method.

In an embodiment, the predetermined time period is calculated according to a determined probability of a blood clot forming within the distal end of the outer device within a defined time period. For example, the determined probability comprises an assessment of one or more of the following factors: a time period since the distal ends of the inner and outer devices were last moved relative to each other; the volume of blood; a measured blood condition of the volume of blood; a measured blood condition of a second volume of blood external to the outer device; and a measured or determined blood coagulation rate of the volume of blood.

In a further embodiment, the measured or determined blood coagulation rate is determined according to one or more of: a separation distance between the distal ends of the inner and outer devices; and the volume of blood.

In an embodiment, the determining step 120 uses of imaging apparatus to determine the position of the inner guidewire and the position of the outer device. In embodiments, the determining step 120 comprises controlling the imaging apparatus to allow the position of the inner guidewire and outer device to be determined. For example, the controlling of the imaging apparatus may comprise movement of a sensor, or adjusting the sensor to allow the position of the inner guidewire and outer device to be determined. For example, a magnification and/or a contrast setting on the sensor may be adjusted, and/or a position of the sensor may be adjusted so that the position of the device may be adjusted.

In an embodiment comprising an imaging apparatus to determine the position of the inner guidewire and outer device, the movement step 130 may comprise adjustments to the imaging apparatus to more accurately determine the relative position of the inner guidewire and outer device. These movements may comprise one or more of a physical movement of the sensor, or an adjustment of one or more settings on the sensor such as a magnification or a contrast setting.

Thus, in an embodiment, in the monitoring step 110 or determination step 110 (or during both steps), an imaging apparatus may comprise a first configuration, and in the movement step 130 the imaging apparatus may comprise a second, different, configuration. For example, during the monitoring step 110 or the determination step 110 the imaging apparatus may comprise a zoomed-out view to provide a broad positional awareness to an operator, and in the movement step 130 (and optional return step 140) the imaging apparatus may comprise a zoomed-in (close-up) view of the catheter so that the precise position of the tips can be accurately determined and moved accordingly.

In an embodiment, a doppler technique is used to measure flow across a vessel.

Alternatively, other empirical measures can be used based on morphology of the anatomy and simulations. Alternatively, digital subtraction angiography can be used to estimate blood flow, wherein fast contrast dissipation implies faster blood flow, and vice versa. According to these methods, a position within the body may be inferred or determined, or a risk of blood clotting may be inferred or determined.

After one cycle of the method is completed, there may be an optional pause before the cycle is repeated in an optional further cycle.

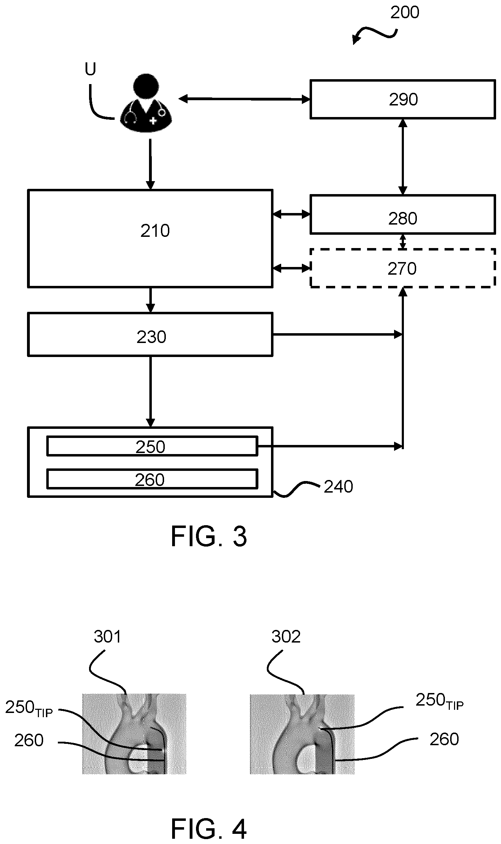

Fig. 3 illustrates a system 200 for controlling a catheter 240 to prevent a clot forming. The system is operated by a user U, who can use a controller 210 to manually position the robot and the catheter. The user U is provided with a GUI and imaging controller 290 of an imaging apparatus 280. The GUI and imaging controller 290 can be used to view the images captured by the imaging apparatus. In use, the imaging apparatus 280 is focused on an area of the patient proximal to where the catheter 240 is positioned. The catheter comprises an inner guidewire 250 (or any other type of inner device 250) and an outer device 260, and the controller 210 controls a driver 130 that moves the inner guidewire 250 and the outer device independently. Thereby the controller 290 can control the driver 130 to change the relative position of the inner guidewire 250 to the outer device 260. Preferably, the inner device 250 and the outer device 260 are elongated and flexible devices.

In an embodiment, the GUI and imaging controller 190 are integrated into the controller 210, and can be used by the user U to interact with the controller 210. In another embodiment, they are separate and the controller has a separate GUI and controls allow the user U to interact with the controller. In embodiments, the controller 210 comprises a notification system.

In an embodiment, the imaging apparatus comprises x ray or ultrasound technology.

In an embodiment, the data from the imaging apparatus 280 is directly used by the controller to determine the relative positions of the inner guidewire 250 and the outer device 260. Any suitable known imaging processing algorithm is used to determine the position of the outer device 260 tip and the position of the inner guidewire 250 tip. One or both of the inner guidewire 250 and outer device 260 may be coated with a special coating (e.g. a metallic coating) to enhance the visibility of the inner guidewire 160 and outer device 260 to make it easier for the algorithm to determine the respective tip positions.

A driver 230 comprises an endovascular robot that drives the devices inside the patient, and which is capable of recording a precise encoding of the relative position of an inner guidewire relative to the outer device. Accordingly, due to the presence of the distance encoding within the driver, the precise relative position of the inner and outer device tips may be known without requiring separate imaging apparatus.

In embodiments, two or more inner guidewires 250 are provided within a catheter 140, controlled by one or more suitable driver 230.

In embodiments, two or more catheters, each comprising one or more inner guidewire 250, and each controlled by a one or more suitable controller 230 are controlled simultaneously by the controller 210.

In an embodiment, an optional robot intelligence module 270 is used to determine the tip positions. This utilizes feedback from the inner guidewire 250 to determine its position relative to the outer device 260. The robot intelligence module 270 comprises a robot encoder that uses signals from the driver 230 to determine the relative position of the tips. In an embodiment, both imaging apparatus 280 and a robot intelligence module 270 are used in tandem to provide position information on the tips.

According to an aspect of this embodiment, the controller 210 and/or 230 is configured to receive (e.g., from the imaging apparatus 280) image data (such image(s) including the inner device 250 and outer device 260) and to receive (e.g., from the endovascular robot) robot or encoding data representative of the driving of the inner device 250 and/or of the outer device 260 by the robot. The controller might further be configured to determine positions of the outer elongated device, to estimate locations of the coaxial inner elongated device. In particular this controller may be further configured to perform this estimation of inner device 250 when it is (fully) retracted within the outer device 260 based on the positions of the outer elongated device and the encoding data, such estimated position of the inner device being optionally indicated graphically on a display displaying the image, as an overlay. This estimation may be implemented once the inner device is retracted within the outer device, based for instance on the determined or estimated position of the tips of the outer device and inner device respectively. Estimation of the distance between the respective tips may further be implemented by the controller in order to better control this implementation, and calibration of the robot data to the pixel data of the images may also help to provide a fusion between the two types of data, in order to output an estimation of the position of the inner device 250 and/or outer device 260 and/or improve the control one or the other of these devices 250-260.

The controller to control the movement of the at least one of the inner device or the outer device responsive to a predetermined condition, wherein the predetermined condition relates to one or more of: a blood parameter of blood proximal to a distal end of the device system; a predetermined time period; and a position of the outer device inside a body of a patient. In an example embodiment the controller 210

Fig. 4 illustrates how imaging data 301, 302 can be used to control the position of the inner guidewire to prevent a clot from forming in a catheter. A set of images 301, 302 are generated by the imaging apparatus 280 of the area of the patient's body where the catheter 240 end is. This set of images may be generated in rapid succession to comprise a video feed. The inner guidewire 250 tip is coated with a suitable material such as metal to make it particularly visible in relation to the surrounding anatomy and to the outer device 260.

The imaging data 301, 302 from the imaging apparatus 280 is provided to the controller 210. An image processing algorithm on the controller 210 is used to determine the relative position of the inner guidewire 250 tip 250_{TIP} relative to the outer device. This information is then used in one or more of monitoring step 110, position determination step 120, movement step 130 or return step 140. For example, at a first time associated with first imaging data 310, it may be determined that a large distance exists between the tip 250_{TIP} of the inner device and the tip of the outer device 260_{TIP}. This information may thus also be used in position determination step 120, since it directly indicates the relative position of the inner guidewire to the outer device. According to this information provided in monitoring step 120 and position determination step 120, the controller 210 may determine that it is necessary to move the inner guidewire to prevent a clot from forming within the end of the catheter 240. The controller 210 sends a signal to the driver 230 to move the inner guidewire relative to the outer device. In movement step second imaging data 302 may be used to determine the new position of the inner guidewire tip 250_{TIP}. Once it has been moved the necessary position, it may be moved back to its original position in optional return step 140, before an optional pause and an optional further cycle of the method.

In embodiments, the operator can configure a time period via the controller, after which the controller may sound an alarm, automatically move the inner device if the time period has been exceeded, or both.

In embodiments, a notification is provided to the user U via the controller 210 if the inner guidewire 250 and outer device 260 tips are separated beyond a threshold distance for longer than a predetermined time period T. In embodiments, the notification may comprise a visual or aural alarm. In embodiments the threshold distance may tend to zero, i.e. be approximately zero.

In embodiments, if the tips are separated by more than a threshold distance for longer than a predetermined time period T, the controller may indicate via a GUI the current risk for clot forming, or a time spent since the tips were last aligned in a tip-to-tip configuration, or both. In embodiments, the risk of clot forming may be determined from empirical data or from mathematical model of the time take for a clot to form, which may be further adjusted according to the separation of the tips. In an embodiment the empirical formula to assess clot forming risk includes one or more of: elapsed time; volume inside catheter; device type/coating; location in the body; and patient characteristics etc., or any combination thereof.

In embodiments, if the amount of time that the inner guidewire tip has been fully inside the catheter and distant from the catheter tip is larger than threshold time T, then automatically advance the tip of the inner guidewire to flush the blood from the end of the catheter. In a variation, the tip may not be advanced until a user U has approved the movement. In embodiments, the repeated automatic flushing is repeated continually, i.e., at fixed intervals, which can help to avoid a clot forming over larger periods of time.

In an embodiment, the inner guidewire continuously oscillates inside the outer device to avoid a clot forming. In an embodiment, the oscillation is periodic. In an embodiment, this oscillation is responsive to a predetermined condition being met.

In an embodiment, the driver controls the position of the outer device 260, and the outer device tip is retraced to the tip of the guidewire, instead of the inner guidewire 250 moving relative to the outer. In a further embodiment, the inner guidewire 250 and the outer device 260 are both moved to adjust the relative distance between the two tips.

In an embodiment, the relative position of the tips is calibrated using imaging. When the tips are aligned, an offset may be defined as zero.

In an embodiment, the tips are aligned before catheter use, and the robot intelligence module 270 is calibrated accordingly. As movement signals are sent by the driver 230 to the inner guidewire and/or outer device, the robot intelligence module 270 determines the new relative positions according to the signal from the driver 230. The controller receives position information from the robot intelligence module and accordingly determines the relative position of the tips of the inner and outer devices. Further, from this relative position information, a volume of blood contained within the end of the outer device can be determined.

In an embodiment, the controller 210 uses the information from the robot intelligence module and/or the imaging apparatus 280 to determine a separation of the tips of the inner and outer devices. Accordingly, a volume of blood therebetween is calculated, based according to the geometry (e.g., cross-sectional area) of the inner and/or outer devices. The controller automatically moves the inner and/or outer tip if a volume of blood exceeds a threshold value. In a further embodiment, this movement is responsive to a threshold time period having been exceeded.

In an embodiment, the controller 210 automatically moves the inner and/or outer device if a predetermined time period has exceeded a threshold. In a further embodiment, this movement is responsive to a distance between the tips and/or a volume of blood therebetween, for example according to an assessment if the distance and/or volume has exceeded a respective predetermined threshold.

In an embodiment, the controller 210 automatically moves the inner guidewire 250 and the outer device 260 to reduce a volume of blood therebetween.

In an embodiment, the controller calculates a determined probability of a blood clot forming within or proximal to the distal end of the outer device within a defined time period, and adjusts the predetermined time period accordingly. For example, if the volume exceeds a threshold, the determined probability may be higher and the time period may be reduced accordingly.

In an embodiment, the determined probability comprises an assessment of one or more of the following factors: a time period since the distal ends of the inner and outer devices were last moved relative to each other; the volume of blood; a measured blood condition of the volume of blood; a measured blood condition of a second volume of blood external to the outer device; and a measured or determined blood coagulation rate of the volume of blood. For example, if the time since the distal ends were last moved exceeds a threshold (3 minutes, for example), the determined probability is increased. As a further example, if the volume of blood exceeds a threshold, the determined probability is increased. As a further example, a measured blood condition of a second volume of blood external to the outer device may change the determined probability - for example, a viscosity of blood outside the outer device may be determined, and if a viscosity threshold is exceeded, the determined probability may be increased. As a further example, if the measured or determined blood coagulation rate of the volume of blood within the end of the outer device exceeds a threshold, the determined probability may be increased.

In an embodiment, the measured or determined blood coagulation rate is determined according to one or more of: a separation distance between the distal ends of the inner and outer devices; and the volume of blood. For example, a function of blood coagulation rate and distal end separation is determined and used to adjust the predetermined time period according to the distal end separation. As a further example a function of blood volume to blood coagulation rate is determined and used to adjust the predetermined time period according to the volume of enclosed blood.

In a further embodiment, the time since the outer device 260 was last moved relative to the body of a patient is assessed. If this time period exceeds a threshold value, the inner and/or outer device may be automatically moved to expel blood from the end of the outer device or to refresh the blood in the outer device.

In a further embodiment, the inner 250 and/or outer 260 device is automatically moved according to at least one of: a relative positioning of the distal end of the inner device with respect to the distal end of the outer device which does not change beyond a positioning change value above an associated temporal value; a change of blood property beyond a threshold value of blood property change; the volume of blood not changing beyond a volume change value above an associated temporal value; the volume of blood over time which not changing enough to prevent a change of blood property beyond a predetermined level of blood property change; and a relative positioning over time of a distal end of the inner device with respect to a distal end of the outer device not changing enough to prevent a change of blood property beyond a predetermined level of blood property change.

In an embodiment, the controller 210, for one or more oscillation, oscillates the position of the inner device 250 or the outer device 260 between a first position relative to the other and a second position relative to the other. For example, the controller 210 is configured to move the inner guidewire 250 from a first position to a second position and back again. In a further embodiment, the controller 210 is configured to continuously move the inner guidewire 250 between a first position and a second position.

In an embodiment the controller 210 controls the movement of the at least one of the inner device 250 and the outer device 260 between a first position of the inner device with respect to the outer device in which the distal end of the inner device is inside the outer device, and a second position of the inner device with respect to the outer device, in which the inner device is in a more retracted position in the first position than in the second position. For example, the controller 210 is configured to move the inner guidewire 250 from a retracted position to a less retracted, wherein in both the retracted position and the less retracted position the tip of the inner guidewire 250 is contained within the outer device 260.

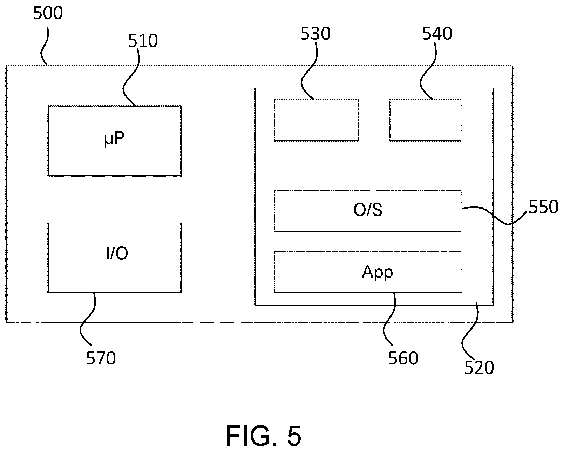

Fig. 5 is a simplified block diagram of a computer within which one or more parts of an embodiment may be employed. Fig. 5 illustrates an example of a computer 500 within which one or more parts of an embodiment may be employed. Various operations discussed above may utilize the capabilities of the computer 500. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520, and one or more I/O devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor-based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 540, source code 530, and one or more applications 560 in accordance with exemplary embodiments. As illustrated, the application 560 comprises numerous functional components for implementing the features and operations of the exemplary embodiments. The application 560 of the computer 500 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. It is contemplated by the inventors that the application 560 for implementing exemplary embodiments may be applicable on all commercially available operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at startup, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, to the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfil the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

Any reference signs in the claims should not be construed as limiting the scope.

## Claims

1. A medical system for controlling a device system within a blood vascular structure, the device system comprising an elongated outer device and an elongated inner device, the inner device being moveable within the outer device, the medical system comprising:
a controller configured to control the movement of at least one of the inner device or the outer device in the lengthwise direction relative to the other,
wherein the controller is further configured to control the movement of the at least one of the inner device or the outer device responsive to a predetermined condition, and
wherein the predetermined condition relates to one or more of: a blood parameter of blood proximal to a distal end of the device system; a lack of mobility of the device system; and blood flow in the area of the vascular structure where the distal end of the device system is located.

2. A medical system for controlling a device system within a blood vascular structure, the device system comprising an elongated outer device and an elongated inner device, the inner device being moveable within the outer device, the medical system comprising:
a controller configured to control the movement of at least one of the inner device or the outer device in the lengthwise direction relative to the other,
wherein the controller is further configured to control the movement of the at least one of the inner device or the outer device responsive to a predetermined condition, and
wherein the predetermined condition relates to clot forming at or proximate a distal end of the device system.

3. A medical system for controlling a device system according to claim 2, wherein the predetermined condition relating to clot forming relates also to one or more of: a blood parameter of blood proximal to a distal end of the outer device; a lack of mobility of the device system; and the location of the distal end of the outer device within the vascular structure.

4. A medical system for controlling a device system according to any of claims 1 to 3, wherein the controller is further configured to control the movement of the at least one of the inner device or the outer device to reduce a volume of blood contained between the distal end of the outer device and the distal end of the inner device.

5. A medical system for controlling a device system according to claim 1 or 3, wherein the lack of mobility is calculated according to a determined probability of a blood clot forming within or proximal to the distal end of the outer device within a defined time period.

6. A medical system for controlling a device system according to claim 5, wherein the determined probability comprises an assessment of one or more of the following factors: a time period since the distal ends of the inner and outer devices were last moved relative to each other; the volume of blood contained between the distal end of the outer device and the distal end of the inner device; a blood condition of the volume of blood; a blood condition of a second volume of blood external to the outer device; and a blood coagulation rate.

7. A medical system for controlling a device system according to claim 6 wherein the blood coagulation rate is determined according to one or more of: a separation distance between the distal ends of the inner and outer devices; and the volume of blood contained between the distal end of the outer device and the distal end of the inner device.

8. A medical system for controlling a device system according to claim 1 or 3, wherein the predetermined condition relating to a predetermined lack of mobility of the device system comprises one or more of:
a relative positioning of the distal end of the inner device with respect to the distal end of the outer device not changing beyond a positioning change value above an associated predetermined time period; and
the volume of blood contained between the distal end of the outer device and the distal end of the inner device not changing beyond a volume change value above an associated predetermined time period;
a predetermined lack of motion of the outer device depending on the location of the outer device with respect to proximate anatomical features.

9. A medical system for controlling a device system according to claim 1 or 3, wherein said predetermined condition relating to a blood parameter of blood comprises one or more of:
a measured or assessed change of blood property beyond a threshold value of blood property change;
the volume of blood contained between the distal end of the outer device and the distal end of the inner device over time not changing enough to prevent a change of blood property beyond a predetermined level of blood property change; and
a relative positioning over time of a distal end of the inner device with respect to a distal end of the outer device not changing enough to prevent a change of blood property beyond a predetermined level of blood property change.

10. A medical system for controlling a device system according to any preceding claim, wherein the controller is further configured to oscillate the position of the inner device or the outer device between a first position and a second position.

11. A medical system for controlling a device system according to any preceding claim, wherein the controller is further configured to control the movement of the at least one of the inner device and the outer device between a first position of the inner device with respect to the outer device in which the distal end of the inner device is inside the outer device, and a second position of the inner device with respect to the outer device, in which the inner device is in a more or less retracted position in the second position than in the first position.

12. A medical system for controlling a device system according to any preceding claim, wherein the controller is configured to identify at least one of a position or a movement of at least one of the inner device and the outer device, according to received images comprising at least at least a portion of the device system, and wherein the controller is configured to control said movement of the at least one of the inner device and the outer device according to the identified position or movement.

13. A medical system for controlling a device system according to any preceding claim, further configured to receive an image comprising at least a portion of the device system and to identify blood clot formation in the image, and wherein the controller is configured to control the movement of at least one of the inner device or the outer device if such blood clot formation fulfills the predetermined condition relating to said blood parameter of blood.

14. A medical system for controlling a device system according to any previous claim, wherein the predetermined condition relating to the blood flow in the area of the vascular structure where the distal end of the device system is located depends on the predetermined lack of motion of the outer device at this location and/or to the distance between the measured or assessed distal end of the outer device with a proximate vessel wall.

15. A computer program product comprising computer program code means which, when executed on a computing device having a processing system, cause a processing system to run a controller of the medical system as recited in any of claims 1 to 14.

16. A controller for use in a medical system for controlling a device system within a blood vasculature structure,
the device system comprising an elongated outer device and an elongated inner device, the inner device being moveable within the outer device,
the controller configured to control the movement of at least one of the inner device or the outer device in the lengthwise direction relative to the other,
wherein the controller is further configured to control the movement of the at least one of the inner device or the outer device responsive to a predetermined condition, and
wherein the predetermined condition relates to one or more of: a blood parameter of blood proximal to a distal end of the device system; a lack of mobility of the device system; and the location of the distal end of the outer device within the vascular structure.
